# EUROPEAN PATENT SPECIFICATION

(11) **EP 4 312 935 B1**
(45) Date of publication and mention of the grant of the patent: **29.01.2025**
(21) Application number: 21715217.2
(22) Date of filing: 24.03.2021
(51) Int. Cl.: A61G 13/10, A61B 90/14

(54) **PATIENT TABLE MOUNTING FRAME**
PATIENTENTISCH-BEFESTIGUNGSRAHMEN
CADRE DE MONTAGE DE TABLE DE PATIENT

(43) Date of publication of application: 07.02.2024
(73) Proprietor: Brainlab AG, 81829 München (DE)
(72) Inventor: HOFBERGER, Stefan, 81829 Munich (DE); PLASSKY, Norman, 81829 Munich (DE)
(74) Representative: SSM Sandmair
(86) International application number: PCT/EP2021/057567
(87) International publication number: WO 2022/199809

(56) References cited:
- EP-A1- 3 644 890
- WO-A1-2016/201418
- US-A1- 2009 308 400
- US-A1- 2015 164 725
- US-B2- 7 238 155

## Description

### FIELD OF THE INVENTION

The present invention relates to mounting systems for attaching medical appliances such as medical robotic systems or medical articulated support arms to a patient table that a patient rests on during a medical procedure. Such a system is disclosed in the us2009308400.

### TECHNICAL BACKGROUND

Robotic assistance plays an increasingly important role in the field of surgery as it improves the capabilities of surgeons, particularly when it comes to high precision surgery and procedures performed on hard to reach parts of a patient's body. Commonly used robotic systems are often provided as standalone systems mounted on a cart for placing the robotic system at a desired position with respect to the patient. In order to ensure that the robotic system remains unaffected from any unintended outside influence such as medical personnel bumping against the cart, those carts are built as sturdy structures which are therefore heavy, cumbersome and require a considerable amount of space that could be used otherwise by medical personnel. Thus, there is an ongoing need for systems to have a small form factor and to integrate into medical procedures more tolerably. Systems are know which connect between a robot and a patient table and therefore exhibit a small footprint. These systems are however at the same time confronted with the problem that widely used interfaces provided by conventional patient tables, such as side-rails of the patient table, are not specifically designed for supporting medical robots and for taking up loads applied by those robots. EP 3 644 890 A1 suggests a head-holder support to be mounted to a patient table, i.e. to side-rails which extend laterally to the patient table, and further includes dedicated sections for receiving a robotic system. The suggested head-holder support overcomes the drawback of previous, space-consuming systems.

It is the object of the present invention to provide a versatile, low-footprint, but still highly robust system for installing a medical robot or similar structure within a medical environment. In particular, it is the object of the present invention to provide a system which is, on the one hand, capable of being mounted to various types of patient tables, and, on the other hand, provides much leeway for installing a medical robot or similar structure at a desired position with respect to the patient.

The present invention can be used for in connection with any system for robot-assisted medical procedures, such as Cirq^{®}, a product of Brainlab AG.

Aspects of the present invention, examples and exemplary steps and their embodiments are disclosed in the following. Different exemplary features of the invention can be combined in accordance with the invention wherever technically expedient and feasible.

### EXEMPLARY SHORT DESCRIPTION OF THE INVENTION

The invention is disclosed in the claims.

The present invention relates to a patient table mounting frame comprising a first frame part and a second frame part coupled to each other via a translatory guide that enables the first frame part and the second frame part to move with respect to each other in a first direction, wherein the first frame part and the second frame part each comprise a table engagement section adapted to releasably connect to a patient table or parts thereof, and wherein at least one of the first frame part and the second frame part includes a mounting section adapted to releasably connect to a corresponding mounting adaptor.

### GENERAL DESCRIPTION OF THE INVENTION

In this section, a description of the general features of the present invention is given for example by referring to possible embodiments of the invention.

The present invention addresses the aforementioned objects by providing a patient table mounting frame that comprises a first and a second frame part which are coupled to each other via a preferably lockable translatory guide that enable the first part and the second part to move with respect to each other in a first direction. The first frame part and the second frame part each comprise a table engagement section that is adapted to releasably connect to a patient table, wherein at least one of the first frame part and the second frame part includes a mounting section adapted to releasably connect to a correspondingly formed mounting adaptor configured to support a medical robot or similar device.

In other words, the inventive mounting frame is basically built as a two-part-structure adapted to receive a patient table between these two frame parts. The two frame parts may engage the patient table or parts thereof from opposite directions, by being moved towards each other along the translatory guide. While both of the two frame parts connect to the patient table, at least one of the frame parts is also adapted to connect to a mounting adaptor which, in some embodiments, does not form part of the present invention. Rather, the mounting adaptor may be provided as an intermediate structure that directly connects to a frame part of the inventive mounting frame on the one hand, and to a surgical robot or similar support structure on the other hand. Further, the mounting adaptor may also be provided as integrated part of the robot or support structure.

Thus, with any configuration of the inventive mounting frame, a first one of the two frame parts may serve as an intermediate structure between the patient table and a surgical robot, that, apart from fastening means, directly connects between the patient table and the robot so as to mount the robot to the patient's table in a most rigid manner. As the number of elements between the table and the robot is thereby reduced to the absolute minimum without any hinges or connecting interfaces between different frame sections that come along with tolerances and plays, the present invention ensures that the spatial position of a robot is accurately maintained with respect to the patient table.

Further, and in any configuration, the second of the two frame parts may serve as an intermediate structure which, apart from fastening means, directly connects between the first frame part and the patient table or parts thereof, thereby reducing the number of structures between the first frame part and an additional connection to the patient table to an absolute minimum, as well.

All in all, the robot is connected to a first section of the patient table via one single frame part, and to a second section of the patient table via both of the frame parts. As the two frame parts can be displaced with respect to each other via the translatory guide, the inventive frame does not only feature a high rigidity, but may also engage various types of patient tables having different dimensions, particularly widths.

In one example, the frame, i.e. both parts thereof may be integrally formed elements and/or may include or be formed from suitable (e.g. radiolucent) material for allowing the frame to be used during various medical imaging procedures including X-Ray-, CT- and/or MR-imaging-procedures, e.g. carbon fibre reinforced plastic.

In a further example, those sections of the first frame part and the second frame part which engage the patient table extend in a direction which is transversal, particularly perpendicular to the direction in which the frame parts are movable with respect to each other.

Moreover, the table engagement sections of the respective frame parts may extend parallel to each other. This allows the mounting frame to engage a certain length of the table or side-rails thereof which extend laterally to the patient's table, wherein the mounting frame may be adapted to various widths of patient tables by moving the frame parts with respect to each other along the translatory guide.

While the mounting frame itself is designed as a most sturdy and rigid support structure, the patient table or parts thereof may be of less sturdiness. In order to get the most stability out of those structures, the inventive mounting frame may engage or connect to the patient table or parts thereof over a predefined distance, particularly over at least 10 cm specifically over at least 20 cm, more specifically over at least 30 cm. By doing so, load applied to the robot acts on large sections of the patient table or parts thereof, thereby reducing possible deformations as much as possible. One can easily imagine that a predefined amount of force or torque will cause less deformation when distributed over a large section of a solid object, as this would be the case when the same amount of force or torque is applied to a smaller section of that object. Thus, the present invention allows for a rigid connection between the patient table and the robot even if certain parts of the patient table are not specifically designed to serve as an interface for connecting robots or similar structures for high-precision medical procedures.

In order for the mounting frame to be connected to various types of patient tables, the table engagement sections of the first frame part and the second frame part may include one or more engagement members, or may connect to one or more engagement members, wherein the one or more engagement members are adapted to releasably engage at least one of
- a side-rail of the patient table which particularly extends laterally of the tabletop of the patient table;
- a frame of the patient table;
- an auxiliary support plate adapted to rest on the upper surface of the tabletop of the patient table.

While the table engagement section of one or both of the frame parts may feature discrete attachment points for one or more engagement members, one or both of the engagement sections may also include sort of an engagement rail or similar structure which allows one or more of the engagement members to connect to a respective engagement section at any desired location along the engagement rail. Thus, positioning the one or more engagement members is not limited by any constraints on the mounting frame side, but may be freely chosen to find the best possible location for connecting to the patient table or parts thereof.

In a further example, the one or more engagement members may releasably connect to the patient table or parts thereof via a friction fit and/or a positive fit. For example, the engagement members may receive a side rail to only allow a translational degree of freedom along the length of the side rail. This remaining degree of freedom may be fixed by a clamping mechanism of the engagement members that establishes a friction fit between the side rail and the corresponding engagement member.

In a further example, the mounting frame includes a locking mechanism configures to selectively fix the position of the first frame part and the second frame part with respect to each other. While this can be done via a positive fit at discrete relative positions of the frame parts, a friction fit allows fixing the frame parts to each other in any desired relative position. The frictional force for maintaining the relative position between the frame parts may for example be significantly increased with the help of "wedge"- or V-shaped sections of the translatory guide, which are pressed against each other via the locking mechanism.

In a further example, the mounting frame comprises a drive mechanism configured to move the first frame part and the second frame part with respect to each other along the translatory guide. The drive mechanism may include one or more actuators or motors, but may also be manually activated, for example via a lever or crank or similar means allowing medical personnel to manually move the frame parts towards each other or away from each other. In this regard, the mounting frame, particularly the engagement sections may be configured to releasably connect to the patient table or parts thereof by being pressed against the patient table or parts thereof via the drive mechanism. In a specific example for connecting the mounting frame to a patient table, the two parts of the mounting frame may be positioned with respect to each other to have an opening for receiving the patient table, which is slightly wider than the actual width of the patient table. After the patient table is received between the two parts of the mounting frame, the drive mechanism may be activated to move both parts of the mounting frame towards each other so as to clamp the patient table therebetween.

In a further example, the first frame part and the second frame part of the mounting frame are adapted to connect to each other along the translatory guide with no intermediate structures therebetween. For example, one of the frame parts may include a cross-sectional extension which is received by a correspondingly shaped cross-section recess of the other one of the frame part. The extension and the recess may have a tapered cross-sectional shape which helps in increasing friction between the two frame parts when, as was previously remarked, the locking mechanism is applied.

While the inventive mounting frame is to provide a most rigid connection between the patient table and a robot or similar structure, the inventive mounting frame may further be formed to exhibit a slim shape that takes away as less space as possible. Thus, the support frame may have a C-shaped geometry with the table engagement sections of the first frame part and the second frame part being spaced apart and extending substantially parallel to each other, wherein the translatory guide extends between respective ends of the engagement sections. This C-shaped geometry allows for positioning the entire support frame underneath a patient table, wherein the table column may be received between the engagement section.

Further, the overall cross-sectional shape of the support frame as seen along a longitudinal axis of the frame and the table may taper in a downward direction. It is then possible for the mounting frame to remain connected to a patient table even when the table is received within a tube shaped opening of a medical imaging device such as an MRI-device.

In a further example, the mounting frame may include one or more mounting adaptors which are to connect to the mounting section of either one of the first frame part and the second frame part. In such example, the mounting adaptor does form part of the inventive mounting frame.

For example, the mounting section may therefore include an engagement rail that enables the one or more mounting adaptors to connect to a respective mounting section at any desired location along the engagement rail. Just like the engagement rails provided for the engagement members that connect to the patient table or parts thereof, the engagement rails provided for the at least one mounting adaptor therefore allow for great flexibility in positioning medical appliances such as a robotic system with respect to the patient table. For example, a robotic system may not only be mounted to the patient table at any desired lateral position along the mounting frame, but may also be mounted either left or right to the patient table, thereby leaving great flexibility for robot-assisted procedures.

In further examples, at least one mounting adaptor is to connect a respective robotic system to the mounting frame. The adaptor may therefore be configured to form part of the robotic system but may also be configured to releasably connect to such robotic system as an intermediate structure between the robotic system and the mounting frame.

Further, it is conceivable to provide at least one mounting adaptor that is formed as a rail extending laterally to and at a distance from one of the table engagement sections. Thus, an auxiliary structure is provided for attaching medical appliances which would normally be attached to the table side-rails which are now occupied by the mounting frame.

In a further example, the mounting frame may further comprise a head-clamp mounting section which is adapted to releasably connect to at least one of the first frame part and the second frame part. The head-clamp mounting section may connect to at least one of the frame parts at any expedient location, for example at a location lateral to a frame part, a location underneath a frame part, a location above a frame part, or a location at the front of a frame part. In particular, the head-clamp mounting section may connect to at least one of the frame parts at a central location adjacent to the translatory guide.

Such head-clamp mounting section may comprise a head-clamp that receives and holds a patient's head in place with respect to the patient table, or may at least comprise an interface for connecting to such head-clamp. Thus, the inventive mounting frame also serves as a support structure for a head-clamp. As the head-clamp connects to the mounting frame with only the head-clamp mounting section as an intermediate structure, the relative position of a patient's head and the robotic system is maintained in a most reliable manner for the benefit of high-precision craniotomy.

The head-clamp mounting section may be formed in the same manner and/or from the same material as the first and the second frame part of the mounting frame, particularly from a radiolucent material. Further, the head-clamp mounting section may connect to the mounting frame at a position underneath the patient table such that no or almost no "overhang" of the patient's head beyond the front edge of the patient table occurs.

In the following, the invention is described with reference to the appended Figures which give background explanations and represent specific embodiments of the invention. The scope of the invention is however not limited to the specific features disclosed in the context of the Figures, wherein
- Figure 1: shows the first and the second frame part as basic components of the inventive mounting frame;
- Figure 2: shows the mounting frame of Figure 1 with additional components of the mounting frame;
- Figure 3: shows the mounting frame of Figure 2 in an assembled state;
- Figure 4: shows a first embodiment of the mounting frame adapted to connect to patient table side-rails;
- Figure 5: shows a second embodiment of the mounting frame adapted to connect directly to a patient table frame;
- Figure 6: shows a third embodiment of the mounting frame adapted to connect to an auxiliary support plate that rests on the tabletop of a patient table.

### DESCRIPTION OF EMBODIMENTS

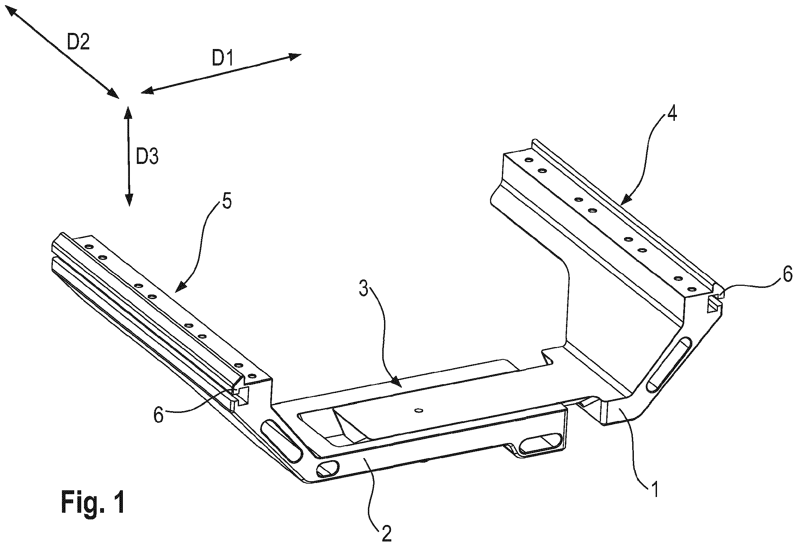

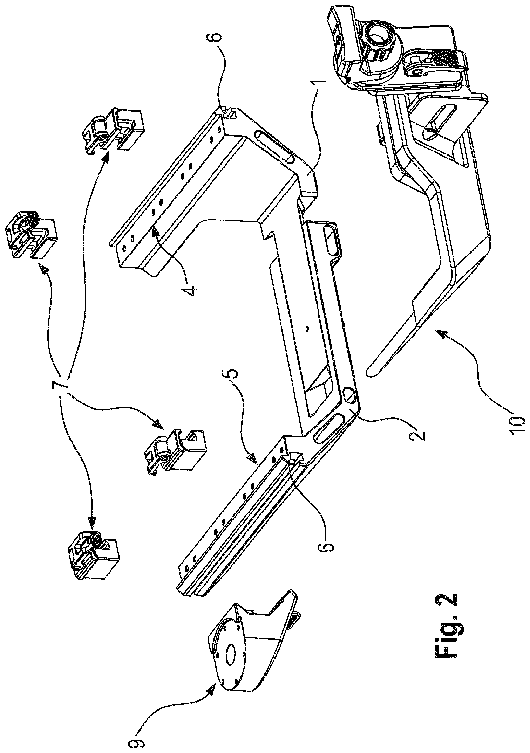

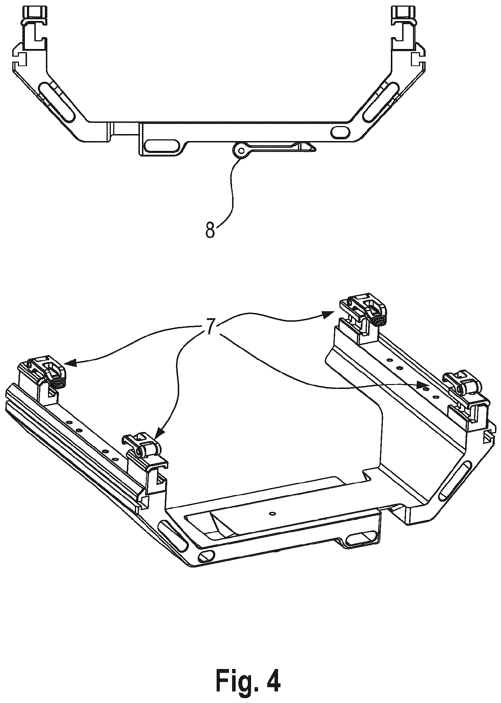

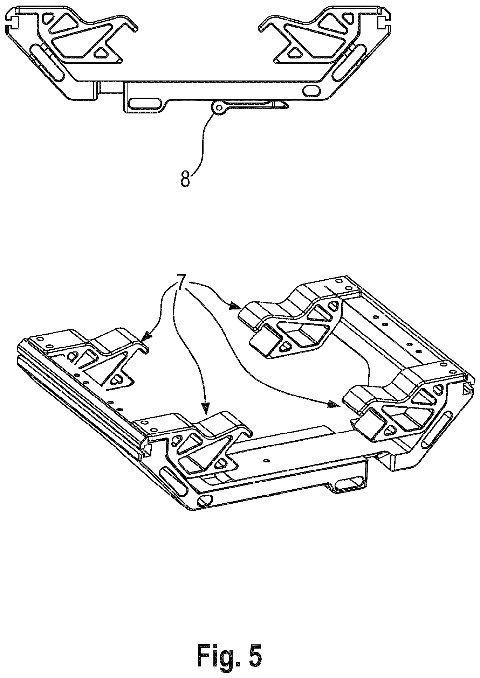

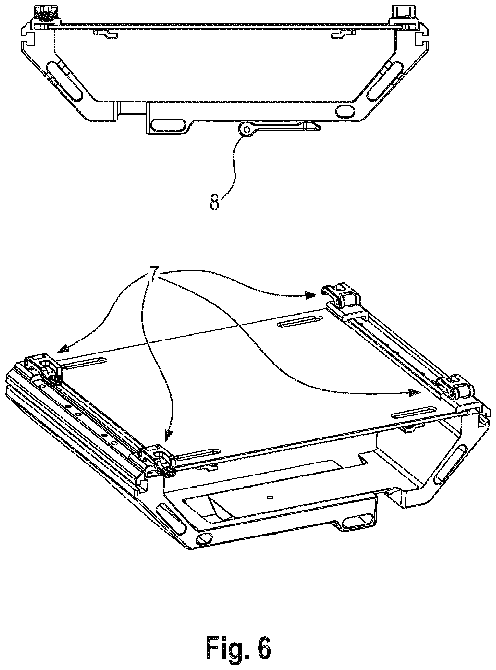

Figure 1 shows the basic part of the inventive mounting frame, namely a first frame part 1 and a second frame part 2 which directly connect each other in a translatory guide 3 that enables the two frame parts 1, 2 to move with respect to each other in a first direction D1. The translatory guide 3 is formed by a wedge shaped extension of the first frame part 1 and a correspondingly shaped recess within the second frame part 2. As soon as the frame parts 1 and 2 are pressed against each other in a direction D3 by a locking mechanism 8 comprising a lever (cf. Figures 4 to 6), this wedge shape increases the frictional force between the frame parts 1 and 2 which reliably holds the frame parts 1 and 2 at a desired position. At both sides of the translatory guide 3, the frame parts 1 and 2 extend in a direction D2, which sections are herein referred to as table engagement sections 4 and 5. In the shown example these engagement sections 4 and 5 include discrete fixation recesses via which engagement members 7 (cf. Figures 2 and 4 to 6) may be attached to the engagement sections 4 and 5 of the respective frame parts 1 and 2.

Further, both of the engagement sections 4 and 5 include a mounting section 6 represented by a rail which extends in the direction D2 and is adapted to receive a mounting adaptor 9 (cf. Figure 2). Both of the frame parts 1 and 2 include recesses for receiving correspondingly shaped extensions of a head-clamp mounting section 10 (cf. Figure 2) at their front faces.

Figure 2 shows multiple components which are adapted to connect to the inventive mounting frame. Figure 2 also shows four engagement members 7 which are configured to engage side-rails of a patient table. Each one of the engagement members 7 comprises a lever-activated clamping mechanism adapted to fix the respective engagement member 7 to a patient table side-rail.

Figure 2 further shows a mounting adaptor 9 adapted to connect between a patient table and a robotic system (such as Cirq^{®}, a product of Brainlab AG, Germany). In the shown example, the mounting adaptor 9 can be mounted to either one of the mounting sections 6 of the first or the second frame part 1, 2, respectively. Thus, the robotic system held by the mounting adaptor 9 can be either positioned left or right to the patient's head that is held via the head clamp mounting section 10 at a central location between the table engagement sections 4 and 5 and the mounting sections 6 thereof.

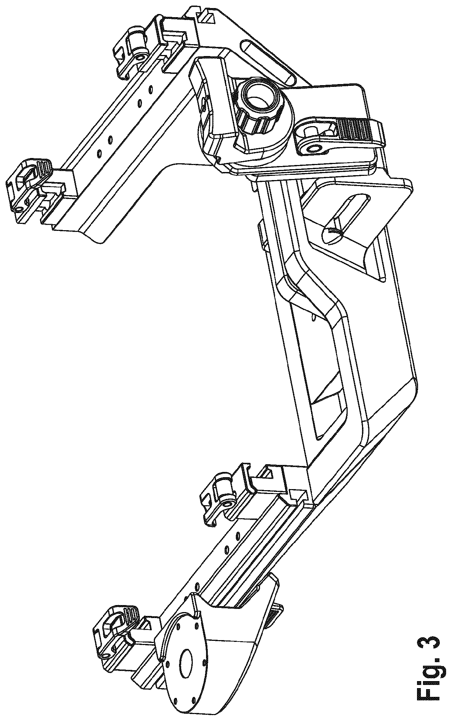

Figure 3 shows the mounting frame of Figure 2 in an assembled state.

As becomes apparent from Figures 4 to 6, various types of engagement members 7 may connect to the frame parts 1 and 2. Thus, the inventive mounting frame can attach to various types of patient tables having different geometries and dimensions, as long as suitably formed engagement members 7 are provided.

## Claims

1. Patient table mounting frame comprising a first frame part (1) and a second frame part (2), and adapted to receive a patient table between these frame parts (1,2) which connect each other along a translatory guide (3) that couples the frame parts (1,2) to each other and enables the first frame part (1) and the second frame part (2) to move with respect to each other in a first direction (D1), wherein the first frame part (1) and the second frame part (2) each comprise a table engagement section (4, 5) adapted to releasably connect to a patient table or parts thereof, and wherein at least one of the first frame part (1) and the second frame part (2) includes a mounting section (6) adapted to provide a releasable connection to a correspondingly formed mounting adaptor (9) which does not form part of the patient table mounting frame.

2. Patient table mounting frame according to claim 1, wherein the table engagement sections (4, 5) of the first frame part (1) and the second frame part (2) extend in a second direction (D2) transversal, particularly perpendicular to the first direction (D1) and/or wherein the table engagement sections (4, 5) of the first frame part (1) and the second frame part (2) extend parallel to each other.

3. Patient table mounting frame according to any one of claims 1 and 2, wherein the table engagement sections (4, 5) of the first frame part (1) and the second frame part (2) are adapted to connect to the patient table or parts thereof over a predefined distance, particularly over at least 10 cm, specifically over at least 20 cm, more specifically over at least 30 cm.

4. Patient table mounting frame according to any one of claims 1 to 3, wherein the table engagement sections (4, 5) of the first frame part (1) and the second frame part (2) each include one or more engagement members (7) adapted to releasably engage at least one of
- a side-rail of the patient table which particularly extends laterally of the tabletop of the patient table;
- a frame of the patient table;
- an auxiliary support plate adapted to rest on the upper surface of the tabletop of the patient table;
particularly wherein at least one of the table engagement sections (4, 5) of the first frame part (1) and the second frame part (2) includes an engagement rail enabling the one or more engagement members (7) to connect to a respective engagement section (4, 5) at a varying location along the engagement rail.

5. Patient table mounting frame according to claim 4, wherein one or more of the engagement members (7) are adapted to releasably connect to the patient table or parts thereof via a friction fit and/or a positive fit established between the one or more engagement members (7) and the patient table or parts thereof, particularly wherein one or more of the engagement members (7) include a clamping mechanism.

6. Patient table mounting frame according to any one of claims 1 to 5, further comprising a locking mechanism (8) adapted to selectively fix the relative position of the first frame part (1) and the second frame part (2), particularly via a friction or positive fit established between the first frame part (1) and the second frame part (2).

7. Patient table mounting frame according to any one of claims 1 to 6, further comprising a drive mechanism adapted to move the first frame part (1) and the second frame part (2) with respect to each other in the first direction (D1), particularly wherein the table engagement sections (4, 5) are adapted to releasably connect to the patient table or parts thereof by being pressed against the patient table or parts thereof via the drive mechanism and/or wherein the drive mechanism is manually activated.

8. Patient table mounting frame according to any one of claims 1 to 7, wherein one of the frame parts (1, 2) includes a cross-sectional extension, specifically a tapered cross-sectional extension, and the other of the frame parts (1, 2) includes a correspondingly shaped cross-sectional recess.

9. Patient table mounting frame according to any one of claims 1 to 8, wherein the patient table support frame has a C-shaped geometry with the table engagement sections (4, 5) of the first frame part (1) and the second frame part (2) being spaced apart and extending substantially parallel to each other, and the translatory guide (3) extending between respective ends of the engagement sections (4, 5).

10. Patient table mounting frame according to any one of claims 1 to 9, wherein the overall cross-sectional shape of the patient table support frame tapers downwards in a third direction (D3) perpendicular to the first direction (D1) and the second direction (D2).

11. Patient table mounting frame according to any one of claims 1 to 10, further comprising at least one mounting adaptor (9) adapted to selectively connect to the mounting section (6) of either one of the first frame part (1) and the second frame part (2).

12. Patient table mounting frame according to any one of claims 1 to 11, wherein the mounting section (6) includes an engagement rail enabling at least one mounting adaptor (9) to connect to a respective mounting section (6) at a varying location along the engagement rail.

13. Patient table mounting frame according to any one of claims 1 to 12, wherein at least one mounting adaptor (9) is configured to form part of or to connect to an automatic or semi-automatic medical robot or articulated support arm.

14. Patient table mounting frame according to any one of claims 1 to 13, wherein at least one mounting adaptor (9) is formed as a rail extending laterally to and at a distance from one of the table engagement sections (4, 5).

15. Patient table mounting frame according to any one of claims 1 to 14, further comprising a head clamp mounting section (10) adapted to releasably connect to at least one of the first frame part (1) and the second frame part (2), particularly at a central location adjacent to the translatory guide (3).

## Patentansprüche

1. Patiententisch-Montagerahmen, der einen ersten Rahmenteil (1) und einen zweiten Rahmenteil (2) umfasst und dazu geeignet ist, einen Patiententisch zwischen diesen Rahmenteilen (1, 2) aufzunehmen, die miteinander entlang einer Translationsführung (3) verbunden sind, die die Rahmenteile (1, 2) miteinander koppelt und es dem ersten Rahmenteil (1) und dem zweiten Rahmenteil (2) ermöglicht, sich relativ zueinander in einer ersten Richtung (D1) zu bewegen, wobei das erste Rahmenteil (1) und das zweite Rahmenteil (2) jeweils einen Tischeingriffsabschnitt (4, 5) aufweisen, der geeignet ist, eine lösbare Verbindung mit einem Patiententisch oder Teilen davon herzustellen, und wobei mindestens eines von dem ersten Rahmenteil (1) und dem zweiten Rahmenteil (2) einen Montageabschnitt (6) aufweist, der geeignet ist, eine lösbare Verbindung mit einem entsprechend ausgebildeten Montageadapter (9) herzustellen, der nicht Teil des Patiententisch-Montagerahmens ist.

2. Patiententisch-Montagerahmen nach Anspruch 1, wobei die Tischeingriffsabschnitte (4, 5) des ersten Rahmenteils (1) und des zweiten Rahmenteils (2) in einer zweiten Richtung (D2) quer, insbesondere senkrecht zur ersten Richtung (D1) verlaufen und/oder wobei die Tischeingriffsabschnitte (4, 5) des ersten Rahmenteils (1) und des zweiten Rahmenteils (2) parallel zueinander verlaufen.

3. Patiententisch-Montagerahmen nach einem der Ansprüche 1 und 2, wobei die Tischeingriffsabschnitte (4, 5) des ersten Rahmenteils (1) und des zweiten Rahmenteils (2) dazu ausgestaltet sind, mit dem Patiententisch oder Teilen davon über einen vorgegebenen Abstand, insbesondere über mindestens 10 cm, insbesondere über mindestens 20 cm, insbesondere über mindestens 30 cm, verbunden zu werden.

4. Patiententisch-Montagerahmen nach einem der Ansprüche 1 bis 3, wobei die Tischeingriffsabschnitte (4, 5) des ersten Rahmenteils (1) und des zweiten Rahmenteils (2) jeweils ein oder mehrere Eingriffselemente (7) aufweisen, die geeignet sind, lösbar in Eingriff zu treten mit mindestens einem von
- einer Seitenschiene des Patiententisches, die sich insbesondere seitlich von der Tischplatte des Patiententisches erstreckt;
- einem Rahmen des Patiententisches;
- einer Hilfs-Aufnahmeplatte, die so ausgestaltet ist, dass sie auf der oberen Fläche der Tischplatte des Patiententisches aufliegt;
insbesondere wobei mindestens einer der Tischeingriffsabschnitte (4, 5) des ersten Rahmenteils (1) und des zweiten Rahmenteils (2) eine Eingriffsschiene aufweist, die es dem einen oder den mehreren Eingriffselementen (7) ermöglicht, mit einem jeweiligen Eingriffsabschnitt (4, 5) an einer variierenden Stelle entlang der Eingriffsschiene verbunden zu werden.

5. Patiententisch-Montagerahmen nach Anspruch 4, wobei eines oder mehrere der Eingriffselemente (7) dazu geeignet sind, mit dem Patiententisch oder Teilen davon über einen Reibschluss und/oder einen Formschluss, der zwischen dem einen oder mehreren Eingriffselementen (7) und dem Patiententisch oder Teilen davon hergestellt wird, lösbar verbunden zu werden, insbesondere wobei eines oder mehrere der Eingriffselemente (7) einen Klemmmechanismus umfassen.

6. Patiententisch-Montagerahmen nach einem der Ansprüche 1 bis 5, ferner umfassend einen Verriegelungsmechanismus (8), der geeignet ist, die relative Position des ersten Rahmenteils (1) und des zweiten Rahmenteils (2) wahlweise festzulegen, insbesondere über einen zwischen dem ersten Rahmenteil (1) und dem zweiten Rahmenteil (2) hergestellten Reib- oder Formschluss.

7. Patiententisch-Montagerahmen nach einem der Ansprüche 1 bis 6, ferner umfassend einen Antriebsmechanismus, der geeignet ist, das erste Rahmenteil (1) und das zweite Rahmenteil (2) relativ zueinander in der ersten Richtung (D1) zu bewegen, insbesondere wobei die Tischeingriffsabschnitte (4, 5) geeignet sind, sich lösbar mit dem Patiententisch oder Teilen davon zu verbinden, indem sie über den Antriebsmechanismus gegen den Patiententisch oder Teile davon gedrückt werden und/oder wobei der Antriebsmechanismus manuell aktiviert wird.

8. Patiententisch-Montagerahmen nach einem der Ansprüche 1 bis 7, wobei eines der Rahmenteile (1, 2) einen Vorsprung im Querschnitt, insbesondere einen verjüngten Vorsprung im Querschnitt, und das andere der Rahmenteile (1, 2) eine entsprechend geformte Ausnehmung im Querschnitt aufweist.

9. Patiententisch-Montagerahmen nach einem der Ansprüche 1 bis 8, wobei der Patiententisch-Montagerahmen eine C-förmige Geometrie aufweist, wobei die Tischeingriffsabschnitte (4, 5) des ersten Rahmenteils (1) und des zweiten Rahmenteils (2) voneinander beabstandet sind und sich im Wesentlichen parallel zueinander erstrecken, und wobei sich die Translationsführung (3) zwischen den jeweiligen Enden der Eingriffsabschnitte (4, 5) erstreckt.

10. Patiententisch-Montagerahmen nach einem der Ansprüche 1 bis 9, wobei sich die Gesamtquerschnittsform des Patiententisch-Montagerahmen in einer dritten Richtung (D3) senkrecht zu der ersten Richtung (D1) und der zweiten Richtung (D2) nach unten verjüngt.

11. Patiententisch-Montagerahmen nach einem der Ansprüche 1 bis 10, der ferner mindestens einen Montageadapter (9) umfasst, der so beschaffen ist, dass er wahlweise mit dem Montageabschnitt (6) entweder des ersten Rahmenteils (1) oder des zweiten Rahmenteils (2) verbunden werden kann.

12. Patiententisch-Montagerahmen nach einem der Ansprüche 1 bis 11, wobei der Montageabschnitt (6) eine Eingriffsschiene aufweist, die es ermöglicht, dass mindestens ein Montageadapter (9) mit einem entsprechenden Montageabschnitt (6) an einer variablen Stelle entlang der Eingriffsschiene verbunden werden kann.

13. Patiententisch-Montagerahmen nach einem der Ansprüche 1 bis 12, wobei mindestens ein Montageadapter (9) so konfiguriert ist, dass er Teil eines automatischen oder halbautomatischen medizinischen Roboters oder eines gelenkigen Stützarms ist oder mit diesem verbunden werden kann.

14. Patiententisch-Montagerahmen nach einem der Ansprüche 1 bis 13, wobei mindestens ein Aufnahmeadapter (9) als Schiene ausgebildet ist, die sich seitlich und mit Abstand zu einem der Tischeingriffsabschnitte (4, 5) erstreckt.

15. Patiententisch-Montagerahmen nach einem der Ansprüche 1 bis 14, ferner mit einem Kopfklammer-Montageabschnitt (10), der zu einer lösbaren Verbindung mit dem ersten Rahmenteil (1) und/oder dem zweiten Rahmenteil (2) ausgestaltet ist, insbesondere an einer zentralen Stelle neben der Translationsführung (3).

## Revendications

1. Cadre de montage de table de patient comprenant une première partie de cadre (1) et une deuxième partie de cadre (2), et adapté pour recevoir une table de patient entre ces parties de cadre (1, 2) qui se raccordent l'une à l'autre le long d'un guide de translation (3) qui couple les parties de cadre (1, 2) l'une à l'autre et permet à la première partie de cadre (1) et à la deuxième partie de cadre (2) de se déplacer l'une par rapport à l'autre dans une première direction (D1), dans lequel la première partie de cadre (1) et la deuxième partie de cadre (2) comprennent chacune une section de mise en prise de table (4, 5) adaptée pour se raccorder de manière libérable à une table de patient ou à des parties de celle-ci, et dans lequel au moins l'une de la première partie de cadre (1) et de la deuxième partie de cadre (2) inclut une section de montage (6) adaptée pour fournir un raccordement libérable à un adaptateur de montage (9) formé correspondant qui ne fait pas partie du cadre de montage de table de patient.

2. Cadre de montage de table de patient selon la revendication 1, dans lequel les sections de mise en prise de table (4, 5) de la première partie de cadre (1) et de la deuxième partie de cadre (2) s'étendent dans une deuxième direction (D2) transversale, en particulier perpendiculaire à la première direction (D1) et/ou dans lequel les sections de mise en prise de table (4, 5) de la première partie de cadre (1) et de la deuxième partie de cadre (2) s'étendent parallèlement l'une à l'autre.

3. Cadre de montage de table de patient selon l'une quelconque des revendications 1 et 2, dans lequel les sections de mise en prise de table (4, 5) de la première partie de cadre (1) et de la deuxième partie de cadre (2) sont adaptées pour se raccorder à la table de patient ou à des parties de celle-ci sur une distance prédéfinie, en particulier sur au moins 10 cm, spécifiquement sur au moins 20 cm, plus spécifiquement sur au moins 30 cm.

4. Cadre de montage de table de patient selon l'une quelconque des revendications 1 à 3, dans lequel les sections de mise en prise de table (4, 5) de la première partie de cadre (1) et de la deuxième partie de cadre (2) incluent chacune un ou plusieurs éléments de mise en prise (7) adaptés pour mettre en prise de manière libérable dans au moins l'un parmi
- un rail latéral de la table de patient qui s'étend en particulier latéralement par rapport au plateau de table de la table de patient ;
- un cadre de la table de patient ;
- une plaque de support auxiliaire adaptée pour reposer sur la surface supérieure du plateau de table de la table de patient ;
en particulier dans lequel au moins l'une des sections de mise en prise de table (4, 5) de la première partie de cadre (1) et de la deuxième partie de cadre (2) inclut un rail de mise en prise permettant aux un ou plusieurs éléments de mise en prise (7) de se raccorder à une section de mise en prise respective (4, 5) à un emplacement variable le long du rail de mise en prise.

5. Cadre de montage de table de patient selon la revendication 4, dans lequel un ou plusieurs des éléments de mise en prise (7) sont adaptés pour se raccorder de manière libérable à la table de patient ou à des parties de celle-ci par l'intermédiaire d'un ajustement par friction et/ou d'un ajustement positif établi entre les un ou plusieurs éléments de mise en prise (7) et la table de patient ou des parties de celle-ci, en particulier dans lequel un ou plusieurs des éléments de mise en prise (7) incluent un mécanisme de serrage.

6. Cadre de montage de table de patient selon l'une quelconque des revendications 1 à 5, comprenant en outre un mécanisme de verrouillage (8) adapté pour fixer sélectivement la position relative de la première partie de cadre (1) et de la deuxième partie de cadre (2), en particulier par l'intermédiaire d'un ajustement par friction ou positif établi entre la première partie de cadre (1) et la deuxième partie de cadre (2).

7. Cadre de montage de table de patient selon l'une quelconque des revendications 1 à 6, comprenant en outre un mécanisme d'entraînement adapté pour déplacer la première partie de cadre (1) et la deuxième partie de cadre (2) l'une par rapport à l'autre dans la première direction (D1), en particulier dans lequel les sections de mise en prise de table (4, 5) sont adaptées pour se raccorder de manière libérable à la table de patient ou à des parties de celle-ci en étant pressées contre la table de patient ou des parties de celle-ci par l'intermédiaire du mécanisme d'entraînement et/ou dans lequel le mécanisme d'entraînement est activé manuellement.

8. Cadre de montage de table de patient selon l'une quelconque des revendications 1 à 7, dans lequel l'une des parties de cadre (1, 2) inclut une extension de section transversale, spécifiquement une extension de section transversale conique, et l'autre des parties de cadre (1, 2) inclut un évidement de section transversale de forme correspondante.

9. Cadre de montage de table de patient selon l'une quelconque des revendications 1 à 8, dans lequel le cadre de support de table de patient présente une géométrie en forme de C, les sections de mise en prise de table (4, 5) de la première partie de cadre (1) et de la deuxième partie de cadre (2) étant espacées et s'étendant sensiblement parallèlement l'une à l'autre, et le guide de translation (3) s'étendant entre des extrémités respectives des sections de mise en prise (4, 5).

10. Cadre de support de table de patient selon l'une quelconque des revendications 1 à 9, dans lequel la forme de section transversale globale du cadre de support de table de patient se rétrécit vers le bas dans une troisième direction (D3) perpendiculaire à la première direction (D1) et à la deuxième direction (D2).

11. Cadre de montage de table de patient selon l'une quelconque des revendications 1 à 10, comprenant en outre au moins un adaptateur de montage (9) adapté pour se raccorder sélectivement à la section de montage (6) de l'une ou l'autre de la première partie de cadre (1) et de la deuxième partie de cadre (2).

12. Cadre de montage de table de patient selon l'une quelconque des revendications 1 à 11, dans lequel la section de montage (6) inclut un rail de mise en prise permettant à au moins un adaptateur de montage (9) de se raccorder à une section de montage (6) respective à un emplacement variable le long du rail de mise en prise.

13. Cadre de montage de table de patient selon l'une quelconque des revendications 1 à 12, dans lequel au moins un adaptateur de montage (9) est configuré pour faire partie d'un robot médical automatique ou semi-automatique ou d'un bras de support articulé ou se raccorder à celui-ci.

14. Cadre de montage de table de patient selon l'une quelconque des revendications 1 à 13, dans lequel au moins un adaptateur de montage (9) est formé comme un rail s'étendant latéralement par rapport à l'une des sections de mise en prise de table (4, 5) et à une distance de celle-ci.

15. Cadre de montage de table de patient selon l'une quelconque des revendications 1 à 14, comprenant en outre une section de montage de pince de tête (10) adaptée pour se raccorder de manière libérable à au moins l'une de la première partie de cadre (1) et de la deuxième partie de cadre (2), en particulier à un emplacement central adjacent au guide de translation (3).
